(19) Europäisches Patentamt
European Patent Office
Office européen des brevets

(11) EP 1 380 567 A1

(12) **EUROPEAN PATENT APPLICATION**

(43) Date of publication:
**14.01.2004 Bulletin 2004/03**

(51) Int Cl.[7]: **C07C 69/94**, C07C 69/90, C09K 19/58

(21) Application number: **03014549.4**

(22) Date of filing: **07.07.2003**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **08.07.2002 JP 2002198697**

(71) Applicant: **MITSUBISHI GAS CHEMICAL COMPANY, INC.**
**Tokyo 100-0005 (JP)**

(72) Inventors:
- **Motoyama, Yuki, Mitsubishi Gas Chemical Co., Inc. Tokyo 125-0051 (JP)**
- **Aoki, Takashi, Mitsubishi Gas Chemical Co., Inc. Tokyo 125-0051 (JP)**
- **Johno, Masahiro, Mitsubishi Gas Chemical Co., Inc. Tokyo 125-0051 (JP)**

(74) Representative: **Albrecht, Thomas, Dr. et al**
**Kraus & Weisert,**
**Thomas-Wimmer-Ring 15**
**80539 München (DE)**

# (54) Chiral compounds as dopants for liquid crystals

(57) An optically active compound of the general formula (1),

$$C_nH_{2n+1}\overset{CH_3}{\overset{|}{C^*}}H\text{-OOC-X-COO-}\overset{CH_3}{\overset{|}{C^*}}HCH_2CH(C_2H_5)_2 \qquad (1)$$

wherein n is an integer of 4 to 8, X is -Ph-COO-Ph-Ph-, -Ph-Ph-COO-Ph-, -Ph-OOC-Ph-Ph-, -Ph-Ph-OOC-Ph-, -Ph-Ph-Ph-, -Cy-COO-Ph-Ph-, -Ph-Ph-OOC-Cy-, -Ph-OOC-Ph-COO-Ph-, -Ph-OOC-Cy-COO-Ph-, -Ph-OOC-Np-COO-Ph-, -Np-OOC-Ph- or -Ph-COO-Np- in which -Ph- is a 1,4-phenylene group, -Cy- is a trans-1,4-cyclohexylene group and -Np- is a 2,6-naphthylene group, and C* is an asymmetric carbon,
and a nematic liquid crystal composition containing the above optically active compound.

According to the present invention, there is provided a nematic liquid crystal composition containing the optically active compound having a helical twisting power (HTP) of 10 or more and giving a chiral dopant for a nematic liquid crystal, which chiral dopant has a property that the pitch of its induced helix decreases in length with an increase in temperature.

EP 1 380 567 A1

## Description

Detailed Description of the Invention

Field of the Invention

The present invention relates to a novel optically active compound useful as a chiral dopant, a liquid crystal composition containing the compound, and a liquid crystal display device to which the liquid crystal composition is applied. More specifically, it relates to a chiral dopant having a helical twisting power (HTP) of at least 10 and having the property of its induced helical pitch decreasing in length with an increase in temperature, and a use thereof.

Prior Art

Various modes are known as display modes of liquid crystal display devices, and in most of such display modes, it is required to control the helical pitch of a liquid crystal. The mode that requires the control of the helical pitch of a liquid crystal includes the following modes.
The modes that have been practically and widely used are a twisted nematic mode (TN mode) and a super twisted nematic mode (STN mode) both using a nematic liquid crystal.
In the TN mode, liquid crystal molecules are aligned so as to twist 90 degrees between an upper substrate and a lower substrate, and a 1/4 pitch of a helix is formed in a cell.
In the STN mode, liquid crystal molecules are aligned so as to twist approximately 220 degrees between an upper substrate and a lower substrate, and an approximately 3/5 pitch of a helix is formed in a cell.
The TN mode is employed in a simple matrix driving liquid crystal display device and an active matrix driving liquid crystal display device, and the STN mode is employed in a simple matrix driving liquid crystal display device.

Brief Description of Drawings

Fig. 1 is a schematic drawing showing a planar alignment state of a chiral nematic liquid crystal.
Fig. 2 is a schematic drawing showing a focal-conic alignment state of a chiral nematic liquid crystal.

Further, there is also a selective reflection (SR) mode of a chiral nematic liquid crystal as another mode in addition to the above TN mode and STN mode. In the SR mode, as shown in Figs. 1 and 2, a liquid crystal has a planar alignment state in which helical axes are perpendicular to substrates (Fig. 1) and a focal-conic alignment state in which directions of helical axes are at random (Fig. 2). These two states are switched from one to the other with voltage pulse. In the planar alignment state, light having a wavelength corresponding to a helical pitch is reflected, and in the focal-conic alignment state, light is transmitted through a device. When a reflection state is used as "bright" and when a transmission state is used as "dark", a display can be materialized.
In the present specification, a "liquid crystal" means a composition containing a plurality of liquid crystal compounds unless it is specified to be a specific compound. Further, a "chiral dopant" means an optical compound that induces a helical structure or a mixture of such a compound. Further, a "base liquid crystal" means a nematic liquid crystal containing no chiral dopant.
As described above, an optically active compound that induces a helical structure is generally called "chiral dopant". Many chiral dopants have been synthesized, and typical compounds thereof are compounds having the following structures.

S811;

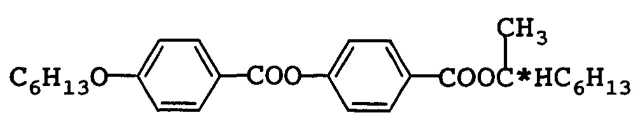

CB15;

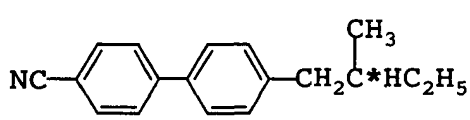

CN; 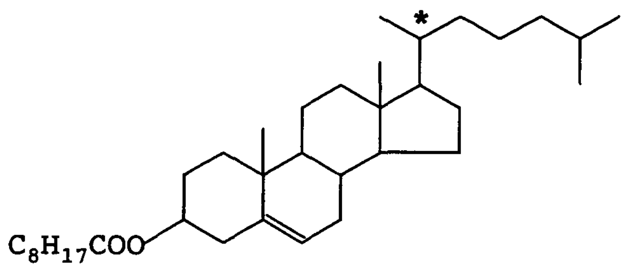

The most essential performance that is required of a chiral dopant is to have large helical twisting power. The helical twisting power (HTP) refers to a physical quantity defined by the following expression.

$$HTP\ (\mu m^{-1}) = 1/(\text{amount of chiral dopant added (wt\%)}/100 \times \text{induced helical pitch } (\mu m))$$

Generally, chiral dopants themselves exhibit no liquid crystallinity, and most of them have large molecular weights. When a large amount of a chiral dopant is added to a base liquid crystal, it degrades various performances in many cases. The degradation of the performances includes a decrease in temperature for phase transition from an isotropic phase to a nematic phase, an increase in viscosity of a liquid crystal and an easy occurrence of crystallization. A chiral dopant having large helical twisting power serves to prevent the degradation of the various performances, since a desired helical pitch can be obtained by adding a small amount of such a chiral dopant to a base liquid crystal.
In addition to the above problems, the SR mode further has a problem that the helical pitch depends upon temperatures. That is, in the SR mode, a liquid crystal reflects (selectively reflects) light corresponding to a helical pitch to generate a bright state. However, when chiral dopants that have been already developed are used, the helical pitch increases in length with an increase in temperature, so that there is caused a problem that reflected light changes in color.
A change in wavelength of selectively reflected light with an increase in temperature is referred to as "wavelength shift". An increase in wavelength of selectively reflected light caused by an increase in temperature is defined to be plus wavelength shift, and a decrease in wavelength of selectively reflected light is defined to be minus wavelength shift.
For removing the dependency of wavelength of selectively reflected light upon temperatures, it has been attempted to combine a chiral dopant that shows a plus wavelength shift and a chiral dopant that shows a minus wavelength shift. However, there are very few chiral dopants that show a minus wavelength shift, and there are reported only four chiral dopants having a helical twisting power (HTP) of at least 9, as are disclosed in U.S. Patent 6217792, JP-A-62-195347 and JP-A-2-053768. Those compounds that have been so far disclosed are not satisfactory, since these compounds exhibit small shift amounts and have a problem that they are liable to cause crystallization even when added in a small amount.
Further, JP-A-02-227489 and JP-A-5-17405 disclose compounds having similar skeleton structures and having molecules containing optically active groups on both of their terminals. The compounds disclosed in these JP Publications have a characteristic feature in a molecular structure. That is, a polar substituent is introduced into their skeleton portions.
The above compounds disclosed have been developed as chiral dopants for a ferroelectric liquid crystal. A chiral dopant for a ferroelectric liquid crystal and a chiral dopant for a nematic liquid crystal greatly differ in performances that they are required to achieve, and the chiral dopant for a ferroelectric liquid crystal cannot be used as a chiral dopant for a nematic liquid crystal.
That is, the chiral dopant for a ferroelectric liquid crystal plays a main role in introducing asymmetry to a liquid crystal composition (generating a ferroelectric property) and inducing spontaneous polarization. Easily polarizing polar substituent (F-, HO-, etc.,) are therefore introduced into its molecule. In a ferroelectric liquid crystal, the addition of a chiral dopant forms a helical structure secondarily, and it is desirable that the length of the helical pitch should be as large as possible. The optically active portion of the chiral dopant is therefore designed so that the power of the chiral dopant to induce a helix is as small as possible.
When the chiral dopant for a ferroelectric liquid crystal, which is designed from the above viewpoint, is used for a nematic liquid crystal, the following problems are caused.

That is, a chiral dopant having a polar substituent decreases the upper limit temperature of a nematic phase to a great extent. In a nematic liquid crystal, further, great importance is attached to the power to induce a helix. A chiral dopant for a ferroelectric liquid crystal is insufficient since it has small helix-inducing power.

Problems to be Solved by the Invention

It is an object of the present invention to provide a chiral dopant having a remarkably large helical twisting power (HTP) of as large as 10 or more and having a property that the pitch of a helix to be induced decreases in length with an increase in temperature.

Means to Solve the Problems

According to the present invention, there is provided an optically active compound of the general formula (1) useful as a chiral dopant,

$$C_nH_{2n+1}\overset{CH_3}{\overset{|}{C^*}}H\text{-OOC-X-COO-}\overset{CH_3}{\overset{|}{C^*}}HCH_2CH(C_2H_5)_2 \qquad (1)$$

wherein n is an integer of 4 to 8, X is -Ph-COO-Ph-Ph-, -Ph-Ph-COO-Ph-, -Ph-OOC-Ph-Ph-, -Ph-Ph-OOC-Ph-, -Ph-Ph-Ph-, -Cy-COO-Ph-Ph-, -Ph-Ph-OOC-Cy-, -Ph-OOC-Ph-COO-Ph-, -Ph-OOC-Cy-COO-Ph-, -Ph-OOC-Np-COO-Ph-, -Np-OOC-Ph- or -Ph-COO-Np- in which -Ph- is a 1,4-phenylene group, -Cy- is a trans-1,4-cyclohexylene group and -Np-is a 2,6-naphthylene group, and C* is an asymmetric carbon.

The optically active compound of the above general formula (1) has excellent properties as a chiral dopant. The above optically active compound is therefore used as an additive to a nematic liquid crystal. That is, at least one member of the above optically active compound is used as a component for a nematic liquid crystal composition.

The above nematic liquid crystal composition is used in a liquid crystal device in which the nematic liquid crystal composition is interposed between substrates having electrodes.

In the above general formula (1), n is an integer of 4 to 8, and n is preferably 5 or 7. Further, X is -Ph-COO-Ph-Ph-. -Ph-Ph-COO-Ph-, -Ph-OOC-Ph-Ph-, -Ph-Ph-OOC-Ph-, -Ph-Ph-Ph-, -Cy-COO-Ph-Ph-, -Ph-Ph-OOC-Cy-, -Ph-OOC-Ph-COO-Ph-, -Ph-OOC-Cy-COO-Ph-, -Ph-OOC-Np-COO-Ph-, -Np-OOC-Ph- or -Ph-COO-Np- in which -Ph- is a 1,4-phenylene group, -Cy- is a trans-1,4-cyclohexylene group and -Np- is a 2,6-naphthylene group. X is preferably -Ph-COO-Ph-Ph-, -Ph-Ph-COO-Ph-, -Ph-OOC-Ph-Ph- or -Ph-Ph-OOC-Ph-.

Further, advantageously, the optically active compound of the above general formula (1) has a helical twisting power (HTP) of 10 or more, and more advantageously, it has an HTP of 14 or more. Preferably, further, the optically active compound of the general formula (1) has a property that the pitch of an induced helix decreases in length with an increase in temperature.

The optically active compound of the present invention has two optically active carbons on the left and right sides as shown in the above general formula (1). The optically active compound therefore includes four optical isomers such as R-R configuration, R-S configuration, S-R configuration and S-S configuration isomers based on the optically active carbons.

Of these optical isomers, R-R configuration and S-S configuration isomers have excellent properties as chiral dopants. The R-R configuration isomer and the S-S configuration isomer differ from each other in twisting direction (right-handed twisting or left-handed twisting) of the helical structure induced. When members of the compound of the general formula (1) are used, they can be therefore selected by taking account of the twisting direction of a chiral dopant to be used in combination.

When a large amount of the optically active compound of the present invention is solely added to a nematic liquid crystal as a base liquid crystal, the resultant composition having some combination may undergo crystallization at room temperature. In this case, however, the crystallization can be easily avoided by using other chiral dopant in combination.

When the optically active compound of the present invention is used as a chiral dopant, the amount of the optically active compound based on the nematic liquid crystal to which the optically active compound is added is generally in the range of 0.1 to 30 % by weight, preferably 0.1 to 20 % by weight. The above amount ratio is preferably determined to be in the above range on the basis of values of helical twisting power (HTP), the crystallinity of the optically active compound and the type of a nematic liquid crystal.

Effect of the Invention

The present invention provides a chiral dopant having a remarkably large HTP of 10 or more, preferably 14 or more, and having a property that the helical pitch induced decreases in length with an increase in temperature. In liquid crystals for use in TN mode or STN mode, therefore, the helical pitch can be adjusted by only adding a small amount of the chiral dopant of the present invention, so that the degradation of performances of a base liquid crystal can be suppressed. In a liquid crystal operated in SR mode, a chiral dopant that induces a plus wavelength shift and the optically active compound of the present invention are used in combination, whereby there can be obtained a liquid crystal composition free of a change that occurs in helical pitch depending upon temperatures.

Examples

The present invention will be more specifically explained with reference to Examples and Comparative Examples hereinafter, while the present invention shall not be limited to these.

Example 1 (General formula (1): n = 5, X = -Ph-COO-Ph-Ph- (E1)), Preparation of 4-((R)-1-methyl-3-ethylpentyloxycarbonyl)biphenyl-4'-((R)-1-methylhexyloxycarbonyl)benzoate

(1) Synthesis of 4'-acetoxybiphenyl-4-carboxylic acid

A reactor was charged with 50 g (234 mmol) of 4'-hydroxybiphenyl-4-carboxylic acid and 238 g (2.34 mol) of acetic anhydride, and 0.1 g of concentrated sulfuric acid was added with stirring. A mixture was stirred until heat generation ceased, and the mixture was further stirred under heat at 80°C for 4 hours. Then, the mixture was gradually cooled to room temperature.

While the above mixture was cooled with an ice bath, 500 g of water was gradually added, and a mixture was stirred at room temperature for 3 hours, to allow unreacted acetic anhydride to be extinct. A precipitated white solid was recovered by filtration, washed with water to remove acetic acid and dried with a vacuum dryer, to give 59.8 g (yield 99 %) of an end compound.

(2) Synthesis of 4'-acetoxybiphenyl-4-carbonyl chloride

A reactor was charged with 59.8 g (233.4 mmol) of 4'-acetoxybiphenyl-4-carboxylic acid and 278 g (2.33 mol) of purified thionyl chloride, and a mixture was refluxed under heat for 4 hours.

Then, thionyl chloride was distilled off under atmospheric pressure, 150 ml of toluene was added to a remainder, and toluene and thionyl chloride were distilled off under reduced pressure, to give 63 g (yield 98 %) of an end compound.

(3) Synthesis of (R)-1-methyl-3-ethylpentyl-4'-acetoxybiphenyl-4-carboxylate

A reactor was charged with 18.6 g (6.7.6 mmol) of 4'-acetoxybiphenyl-4-carbonyl chloride, 8.0 g (61.4 mmol) of (R)-4-ethyl-2-hexanol and 140 ml of toluene, 9.7 g (122.9 mmol) of pyridine was dropwise added thereto, and a mixture was stirred at room temperature for 3 hours.

To a reaction solution was added 40 ml of water, a mixture was stirred at room temperature for 30 minutes, and then, a liquid of an organic layer was separated.

The organic layer was washed with 2N hydrochloric acid, with a 1N sodium hydroxide aqueous solution and with water, and the washed organic layer was dried over anhydrous sodium sulfate and filtered. Then, the solvent was distilled off, to give 22 g (yield 97 %) of an end compound.

(4) Synthesis of (R)-1-methyl-3-ethylpentyl-4'-hydroxybiphenyl-4-carboxylate

A reactor was charged with 22 g (59.7 mmol) of (R)-1-methyl-3-ethylpentyl-4'-acetoxybiphenyl-4-carboxylate and 390 ml of toluene, a methanol solution containing 40 % methylamine was dropwise added, and a mixture was stirred at room temperature for 3 hours.

A reaction solution was washed with 2N hydrochloric acid and with water, to separate the solution, and an organic layer was dried over anhydrous sodium sulfate and filtered. Then, the solvent was distilled off, to give 19 g (yield 97 %) of an end compound.

(5) Synthesis of 4-((R)-1-methyl-3-ethylpentyloxycarbonyl)biphenyl-4'-((R)-1-methylhexyloxycarbonyl)benzoate

A reactor was charged with 2.0 g (6.12 mmol) of (R)-1-methyl-3-ethylpentyl-4'-hydroxybiphenyl-4-carboxylate, 1.8 g (8.86 mmol) of terephthalyl dichloride and 150 ml of dehydrated dichloromethane, 3 g (37 mmol) of pyridine was dropwise added, and a mixture was stirred at room temperature for 6 hours. To the mixture was further added 1.3 g (11.5 mmol) of (R)-2-heptanol, and the mixture was stirred at room temperature for 18 hours. To the thus-obtained reaction solution was added 50 ml of water, and a mixture was stirred at room temperature for 2 hours. Then, a liquid of an organic layer was separated. The organic layer was washed with 2N hydrochloric acid, with a 1N sodium hydroxide aqueous solution and then with water, and the washed organic layer was dried over anhydrous sodium sulfate and filtered. Then, the solvent was distilled off.

The thus-obtained crude product was purified by silica gel chromatography, to give 1.6 g (2.75 mmol, yield 31 %) of an end compound.

Example 2 (General formula (1): n = 5, X = -Ph-Ph-OOC-Ph- (E2)), Preparation of 4-((R)-1-methylhexyloxycarbonyl) biphenyl-4'-((R) -1-methyl-3-ethylpentyloxycarbonyl)benzoate

(1) Synthesis of (R)-1-methylheyxl-4'-hydroxybiphenyl-4-carboxylate

The above end compound was obtained in the same manner as in the procedures (1) to (4) of Example 1 except that (R)-4-ethyl-2-hexanol was replaced with (R)-2-heptanol in the procedure of (3) of Example 1.

(2) Synthesis of 4-((R)-1-methylhexyloxycarbonyl)biphenyl-4'-((R)-1-methyl-3-ethylpentyloxycarbonyl)benzoate

A reactor was charged with 1.9 g (6.12 mmol) of (R)-1-methylhexyl-4'-hydroxybiphenyl-4-carboxylate, 1.8 g (8.86 mmol) of terephthalyl dichloride and 150 ml of dehydrated dichloromethane, 3 g (37 mmol) of pyridine was dropwise added, and a mixture was stirred at room temperature for 6 hours. To the mixture was further added 1.5 g (11.5 mmol) of (R)-4-ethyl-2-hexanol, and the mixture was stirred at room temperature for 18 hours. To the thus-obtained reaction solution was added 50 ml of water, and a mixture was stirred at room temperature for 2 hours. Then, a liquid of an organic layer was separated. The organic layer was washed with 2N hydrochloric acid, with a 1N sodium hydroxide aqueous solution and then with water, and the washed organic layer was dried over anhydrous sodium sulfate and filtered. Then, the solvent was distilled off.

The thus-obtained crude product was purified by silica gel chromatography, to give 1.4 g (2.48 mmol, yield 28 %) of an end compound.

Example 3 (General formula (1): n = 5, X = -Ph-Ph-COO-Ph- (E3)), Preparation of 4-((R)-1-methyl-3-ethylpentyloxycarbonyl)phenyl-4'-((R)-1-methylhexyloxycarbonyl)biphenyl-4-carboxylate

(1) Synthesis of 4-acetoxybenzoyl chloride

100 g (0.555 mol) of 4-acetoxybenzoic acid was added to 400 g (3.36 mol) of thionyl chloride, and a mixture was refluxed under heat for 4 hours. Then, excess thionyl chloride was distilled off, and a remainder was purified by distillation under reduced pressure (4 mmHg, 116°C), to give 99 g (0.498 mol, yield 90 %) of an end compound.

(2) Synthesis of (R)-4-acetoxy-1-(1-methyl-3-ethylpentyloxycarbonyl)benzene

A reactor was charged with 49 g (0.246 mol) of 4-acetoxybenzoyl chloride, 29 g (0.222 mol) of (R)-4-ethyl-2-hexanol and 750 ml of dehydrated toluene, 35 g (0.442 mol) of pyridine was dropwise added thereto, and a mixture was stirred at room temperature for 15 hours. To a reaction solution was added 300 ml of water, a mixture was stirred at room temperature for 1 hour, and then, a liquid of an organic layer was separated. The organic layer was washed with 2N hydrochloric acid, with a 1N sodium hydroxide aqueous solution and with water.

The washed organic layer was dried over anhydrous sodium sulfate and filtered. Then, the solvent was distilled off, to give 63 g (0.215 mol, yield 97 %) of an end compound.

(3) Synthesis of (R)-4-hydroxy-1-(1-methyl-3-ethylpentyloxycarbonyl)benzene

63 g (0.215 mol) of (R)-4-acetoxy-1-(1-methyl-3-ethylpentyloxycarbonyl)benzene was dissolved in 900 ml of dehydrated toluene, 25 g of a methanol solution containing 40 % of methylamine was dropwise added thereto, and a mixture was stirred at room temperature for 5 hours. A reaction solution was washed with 2N hydrochloric acid and

with water. An organic layer was dried over anhydrous sodium sulfate and filtered, and then the solvent was distilled off, to give 49 g (0.195 mol, yield 91 %) of an end compound.

(4) Synthesis of 4-((R)-1-methyl-3-ethylpentyloxycarbonyl)phenyl-4' -((R)-1-methylhexyloxycarbonyl)biphenyl-4-carboxylate

A reactor was charged with 3.1 g (12.3 mmol) of (R)-4-hydroxy-1-(1-methyl-3-ethylpentyloxycarbonyl)benzene, 3.1 g (11.1 mmol) of 4,4'-biphenyldicarbonyl chloride and 100 ml of dehydrated dichloromethane, 4 g (48 mmol) of pyridine was dropwise added, and a mixture was stirred at room temperature for 6 hours. To the mixture was further added 1.3 g (11.5 mmol) of (R)-2-heptanol, and the mixture was stirred at room temperature for 18 hours. To the thus-obtained reaction solution was added 50 ml of water, and a mixture was stirred at room temperature for 2 hours. Then, a liquid of an organic layer was separated. The organic layer was washed with 2N hydrochloric acid, with a 1N sodium hydroxide aqueous solution and then with water, and the washed organic layer was dried over anhydrous sodium sulfate and filtered. Then, the solvent was distilled off.

The thus-obtained crude product was purified by silica gel chromatography, to give 1.9 g (3.34 mmol, yield 30 %) of an end compound.

Example 4 (General formula (1): n = 5, X = -Ph-OOC-Ph-Ph- (E4)), Preparation of 4-((R)-1-methylhexyloxycarbonyl) phenyl-4'-((R)-1-methyl-3-ethylpentyloxycarbonyl)biphenyl-4-carboxylate

(1) Synthesis of (R)-4-hydroxy-1-(1-methylhexyloxycarbonyl)benzene

The above end compound was obtained in the same manner as in the procedures (1) to (3) of Example 3 except that (R)-4-ethyl-2-hexanol was replaced with (R)-2-heptanol in the procedure (2) of Example 3.

(2) Synthesis of 4-((R)-1-methylhexyloxycarbonyl)phenyl-4'-((R)-1-methyl-3-ethylpentyloxycarbonyl)biphenyl-4-carboxylate

A reactor was charged with 2.9 g (12.3 mmol) of (R)-4-hydroxy-1- (1-methylhexyloxycarbonyl)benzene, 3.1 g (11.1 mmol) of 4,4'-biphenyldicarbonyl chloride and 100 ml of dehydrated dichloromethane, 4 g (48 mmol) of pyridine was dropwise added, and a mixture was stirred at room temperature for 6 hours. To the mixture was further added 1.5 g (11.5 mmol) of (R)-4-ethyl-2-hexanol, and the mixture was stirred at room temperature for 18 hours. To the thus-obtained reaction solution was added 50 ml of water, and a mixture was stirred at room temperature for 2 hours. Then, a liquid of an organic layer was separated. The organic layer was washed with 2N hydrochloric acid, with a 1N sodium hydroxide aqueous solution and then with water, and the washed organic layer was dried over anhydrous sodium sulfate and filtered. Then, the solvent was distilled off.

The thus-obtained crude product was purified by silica gel chromatography, to give 2.1 g (3.66 mmol, yield 33 %) of an end compound.

The optically active compounds (E1 to E4) obtained in the above Examples 1 to 4 were measured for thermal properties by DSC (temperature elevation). The results were as below.

E1: Cry(<-20)Iso    E2: Cry(<-20)Iso

E3: Cry(59)Iso    E4: Cry(54)Iso

In the above results, parenthesized values show phase transfer temperatures (°C), Iso stands for an isotropic phase, and Cry stands for a crystal phase.

Structural formulae of common portion and characteristic portions (A portion) of the optically active compounds (E1 to E4) are shown below. Table 1 shows $^1$H-NMR measurement results of these compounds.

Common portion

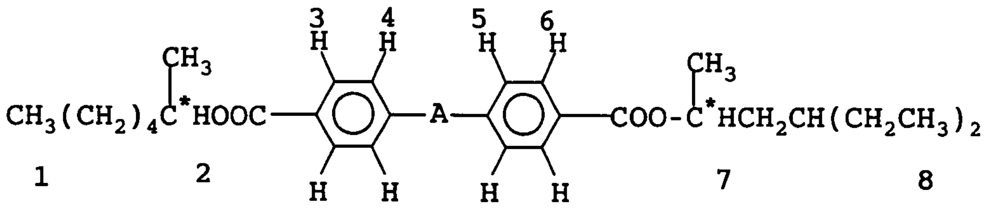

A portion

[E1]

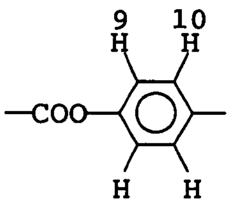

[E2]

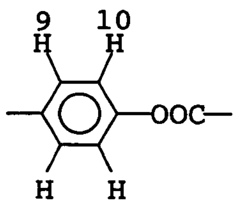

[E3]

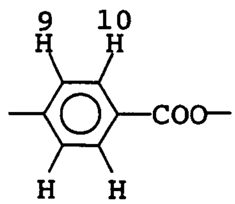

[E4]

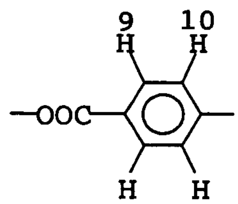

Table 1

$^1$H-NMR (δ, ppm)

| No. | Common portion | | | | | | | | A portion | |
|---|---|---|---|---|---|---|---|---|---|---|
| | 1 | 2 | 3 | 4 | 5 | 6 | 7 | 8 | 9 | 10 |
| (E1): | 0.88 | 5.18-5.28 | 8.18 | 8.28 | 7.68 | 8.11 | 5.18-5.28 | 0.88 | 7.34 | 7.68 |
| (E2): | 0.88 | 5.16-5.21 | 8.12 | 7.68 | 8.29 | 8.18 | 5.16-5.21 | 0.88 | 7.68 | 7.34 |
| (E3): | 0.88 | 5.20-5.25 | 8.15 | 7.72 | 7.32 | 8.15 | 5.20-5.25 | 0.88 | 7.77 | 8.30 |
| (E4): | 0.87 | 5.15-5.20 | 8.15 | 7.32 | 7.73 | 8.15 | 5.15-5.20 | 0.87 | 8.30 | 7.77 |

Example 5

The above-prepared optically active compounds (E1 to E4) were measured for helical twisting powers (HTP) and wavelength shifts.

To a nematic liquid crystal (ZLI-1565) supplied by Merck & Co., Inc., was added 10 % by weight, based on a

composition, of the optically active compound (E1) obtained in Example 1, to prepare a chiral nematic (N*) liquid crystal composition.

The thus-prepared liquid crystal composition was measured for an upper-limit temperature of its N* phase and selective reflection behaviors, and its helical twisting power (HTP) was determined on the basis of the selective reflection behaviors.

The upper-limit temperature of the N* phase was determined by observation through a polarizing microscope.

Further, the selective reflection behaviors were measured according to the following procedures.

A liquid crystal cell with ITO electrodes (cell thickness 10 μm) was charged with the above-prepared liquid crystal composition in an isotropic state. The cell was adjusted to 60°C, a rectangular wave voltage of ±60 V was applied for approximately 1 minute, and the cell was rapidly cooled to room temperature to attain planar alignment.

The above liquid crystal cell was evaluated for selective reflection behaviors at 25°C and 60°C with an automatic spectrophotometer. HTPs at 25°C and 60°C were calculated on the basis of the following expressions.

$$\text{HTP } (\mu m^{-1}) = n/(\lambda_{25} \text{ x } C/100)$$

$$\text{HTP } (\mu m^{-1}) = n/(\lambda_{6o} \text{ x } C/100)$$

wherein n is a refractive index of the chiral nematic liquid crystal, $\lambda_{25}$ is a selective reflection wavelength (μm) at 25°C, $\lambda_{60}$ is a selective reflection wavelength (μm) at 60°C, and C is a concentration (wt%) of the optically active compound. As a refractive index n, there was employed a value of 1.6 that ZLI-1565 as a base liquid crystal had.

The wavelength shift was determined on the basis of the following expression.

$$\text{Wavelength shift (nm)} = \lambda_{60}^{*} - \lambda_{25}^{*}$$

wherein $\lambda_{60}$* is a selective reflection wavelength (nm) at 60°C and $\lambda_{25}$* is a selective reflection wavelength (nm) at 25°C.

Table 2 shows the results. The optically active compound (E1) of Example 1 had a large HTP of 10 or more and had a property that the induced helical pitch decreased in length with an increase in temperature.

The optically active compounds (E2 to E4) obtained in Examples 2 to 4 were measured for upper-limit temperatures of their N* phases, HTPs and wavelength shifts in the same manner as above. Table 2 shows the results.

Comparative Examples 1 to 3

Those known optically active compounds CB15, S811 and CN explained in the description of Prior Art were measured for upper-limit temperatures of their N* phases, HTPs and wavelength shifts in the same manner as in Example 5.

CB15 and S811 were added in an amount of 15 % by weight each on the basis of the nematic liquid crystal, CN was added in an amount of 30 % by weight on the same basis, and the resultant compositions were measured for helical twisting powers (HTP). Table 2 shows the results.

Table 2

| Compound | Iso-N*(°C) | HTP(1/μm) | Wavelength shift(nm) |
|---|---|---|---|
| E1 | 82 | 15.2 | -108 |
| E2 | 82 | 15.4 | -109 |
| E3 | 82 | 16.0 | -84 |
| E4 | 81 | 15.6 | -100 |
| CB15 | 74 | 7.9 | +193 |
| S811 | 73 | 10.1 | +7 |
| CN | 82 | 5.2 | +34 |

Note) Iso-N* shows a phase transition temperature in a transition from an isotropic phase to a chiral nematic phase (upper-limit temperature of N* phase).

## Claims

1. An optically active compound of the general formula (1),

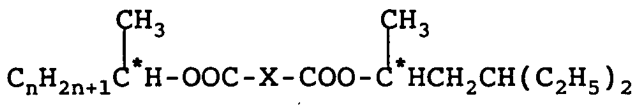

(1)

wherein n is an integer of 4 to 8, X is -Ph-COO-Ph-Ph-, -Ph-Ph-COO-Ph-, -Ph-OOC-Ph-Ph-, -Ph-Ph-OOC-Ph-, -Ph-Ph-Ph-, -Cy-COO-Ph-Ph-, -Ph-Ph-OOC-Cy-, -Ph-OOC-Ph-COO-Ph-, -Ph-OOC-Cy-COO-Ph-, -Ph-OOC-Np-COO-Ph-, -Np-OOC-Ph- or -Ph-COO-Np- in which -Ph- is a 1,4-phenylene group, -Cy- is a trans-1,4-cyclohexylene group and -Np-is a 2,6-naphthylene group, and C* is an asymmetric carbon.

2. The optically active compound of claim 1, which has the general formula (1) in which n is 5 or 7.

3. The optically active compound of claim 1, which has the general formula (1) in which X is -Ph-COO-Ph-Ph-, -Ph-Ph-COO-Ph-, -Ph-OOC-Ph-Ph- or -Ph-Ph-OOC-Ph-.

4. The optically active compound of claim 1, which has a helical twisting power (HTP) of 10 or more.

5. The optically active compound of claim 1, which induces a helical pitch and has a property that the induced helical pitch decreases in length with an increase in temperature.

6. The optically active compound of claim 1, wherein two asymmetric carbons shown in the general formula (1) are R-configuration isomers together or S-configuration isomers together.

7. A chiral dopant of the general formula (1) in claim 1 for a nematic liquid crystal.

8. A nematic liquid crystal composition containing at least one member compound of the optically active compound of the general formula (1) in claim 1.

9. A liquid crystal display device having the nematic liquid crystal composition recited in claim 8 interposed between substrates having an electrode each.

F I G. 1

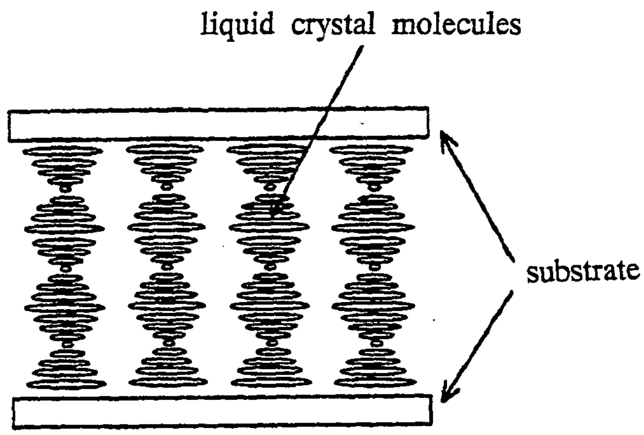
liquid crystal molecules
substrate

F I G. 2

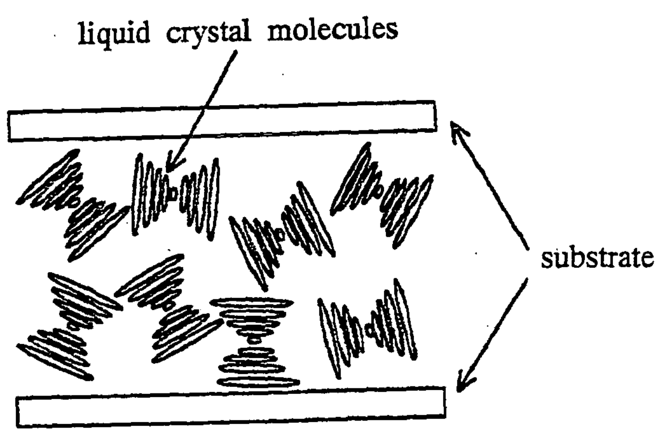
liquid crystal molecules
substrate

European Patent Office

# EUROPEAN SEARCH REPORT

Application Number
EP 03 01 4549

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| D,A | PATENT ABSTRACTS OF JAPAN vol. 017, no. 291 (C-1067), 4 June 1993 (1993-06-04) & JP 05 017405 A (KANEBO LTD), 26 January 1993 (1993-01-26) * abstract * --- | 1-9 | C07C69/94 C07C69/90 C09K19/58 |
| D,A | PATENT ABSTRACTS OF JAPAN vol. 014, no. 539 (C-0782), 28 November 1990 (1990-11-28) & JP 02 227489 A (DAINIPPON INK & CHEM INC;OTHERS: 01), 10 September 1990 (1990-09-10) * abstract * --- | 1-9 | |
| A | PATENT ABSTRACTS OF JAPAN vol. 017, no. 309 (C-1070), 14 June 1993 (1993-06-14) & JP 05 025085 A (KASHIMA SEKIYU KK), 2 February 1993 (1993-02-02) * abstract * --- | 1-9 | TECHNICAL FIELDS SEARCHED (Int.Cl.7) |
| A | NAKAUCHI J ET AL: "FERROELECTRIC LIQUID CRYSTAL MIXTURES DOPED WITH COMPOUNDS HAVING CHIRAL GROUPS AT BOTH ENDS OF THE CORE" JAPANESE JOURNAL OF APPLIED PHYSICS, PUBLICATION OFFICE JAPANESE JOURNAL OF APPLIED PHYSICS. TOKYO, JP, vol. 28, no. 2, 1 February 1989 (1989-02-01), pages L272-274, XP000098738 ISSN: 0021-4922 * the whole document * --- | 1-9 | C07C C09K |
| P,A | EP 1 270 542 A (MITSUBISHI GAS CHEMICAL CO.) 2 January 2003 (2003-01-02) * the whole document * --- -/-- | 1-9 | |

The present search report has been drawn up for all claims

| Place of search | Date of completion of the search | Examiner |
|---|---|---|
| THE HAGUE | 12 September 2003 | Beslier, L |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

European Patent Office

# EUROPEAN SEARCH REPORT

Application Number
EP 03 01 4549

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| P,A | EP 1 249 484 A (MITSUBISHI GAS CHEMICAL CO.) 16 October 2002 (2002-10-16)<br>* the whole document *<br>----- | 1-9 | |

TECHNICAL FIELDS SEARCHED (Int.Cl.7)

The present search report has been drawn up for all claims

| Place of search | Date of completion of the search | Examiner |
|---|---|---|
| THE HAGUE | 12 September 2003 | Beslier, L |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 03 01 4549

This annex lists the patent family members relating to the patent documents cited in the above-mentioned European search report.
The members are as contained in the European Patent Office EDP file on
The European Patent Office is in no way liable for these particulars which are merely given for the purpose of information.

12-09-2003

| Patent document cited in search report | | Publication date | Patent family member(s) | | | Publication date |
|---|---|---|---|---|---|---|
| JP 05017405 | A | 26-01-1993 | NONE | | | |
| JP 02227489 | A | 10-09-1990 | NONE | | | |
| JP 05025085 | A | 02-02-1993 | NONE | | | |
| EP 1270542 | A | 02-01-2003 | JP | 2003012610 | A | 15-01-2003 |
| | | | EP | 1270542 | A1 | 02-01-2003 |
| | | | US | 2003124268 | A1 | 03-07-2003 |
| EP 1249484 | A | 16-10-2002 | JP | 2002308833 | A | 23-10-2002 |
| | | | JP | 2002322135 | A | 08-11-2002 |
| | | | CN | 1381438 | A | 27-11-2002 |
| | | | EP | 1249484 | A1 | 16-10-2002 |
| | | | US | 2003054119 | A1 | 20-03-2003 |

EPO FORM P0459

For more details about this annex : see Official Journal of the European Patent Office, No. 12/82